(19) Europäisches Patentamt
European Patent Office
Office européen des brevets

(11) Veröffentlichungsnummer : **0 334 035 B1**

# (12) EUROPÄISCHE PATENTSCHRIFT

(45) Veröffentlichungstag der Patentschrift : **21.11.91 Patentblatt 91/47**

(51) Int. Cl.$^5$ : **C07D 233/58**, C07D 249/08, C07D 405/06, A61K 31/41, A61K 31/415

(21) Anmeldenummer : **89103025.6**

(22) Anmeldetag : **21.02.89**

## (54) Azolylpropenyl- und Azolylmethyloxiran-Derivate und diese enthaltende Fungizide.

(30) Priorität : **26.02.88 DE 3806089**

(43) Veröffentlichungstag der Anmeldung : **27.09.89 Patentblatt 89/39**

(45) Bekanntmachung des Hinweises auf die Patenterteilung : **21.11.91 Patentblatt 91/47**

(84) Benannte Vertragsstaaten : **AT BE CH DE ES FR GB GR IT LI NL SE**

(56) Entgegenhaltungen :
**EP-A- 0 150 892**
**EP-A- 0 196 038**
**EP-A- 0 255 243**
**DE-A- 2 549 798**
**DE-A- 2 652 313**
**DE-A- 2 750 031**
**DE-A- 3 218 129**
**DE-A- 3 218 130**
**DE-A- 3 511 411**

(56) Entgegenhaltungen :
**CHEMICAL ABSTRACTS, Band 93, Nr. 23, 8. Dezember 1980, Columbus, Ohio, USA; RAAB, W. et al.: "Antifungal activity of imidazole derivatives" Seite 102, Spalte 1, Zusammenfassung-Nr. 215 969d**
**CHEMICAL ABSTRACTS, Band 99, Nr. 23, 5. Dezember 1983, Columbus, Ohio, USA; STOTSKII, A.A. et al.: "Synthesis and tranformations of N-alkenyl-1,2,4-triazoles" Seite 748, Spalte 2, Zusammenfassung-Nr. 194 892e**

(73) Patentinhaber : **BASF Aktiengesellschaft**
**Carl-Bosch-Strasse 38**
**W-6700 Ludwigshafen (DE)**

(72) Erfinder : **Seele, Rainer, Dr.**
**Leiblstrasse 3**
**W-6701 Fussgoenheim (DE)**
Erfinder : **Karbach, Stefan, Dr.**
**Grundwiesenweg 44**
**W-6730 Neustadt (DE)**
Erfinder : **Goetz, Norbert, Dr.**
**Schoefferstrasse 25**
**W-6520 Worms 1 (DE)**
Erfinder : **Lorenz, Gisela, Dr.**
**Erlenweg 13**
**W-6730 Neustadt (DE)**
Erfinder : **Ammermann, Eberhard, Dr.**
**Sachsenstrasse 3**
**W-6700 Ludwigshafen (DE)**

**EP 0 334 035 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

## Beschreibung

Die vorliegende Erfindung betrifft neue Azolverbindungen, Verfahren zu ihrer Herstellung und diese enthaltende Fungizide, sowie Verfahren zur Bekämpfung von Pilzen mit diesen Verbindungen.

Es ist bekannt, Azolylmethyloxirane z.B. das 2-(1,2,4-Triazol-1-yl-methyl)-2-(tert.-butyl)-3-(4-chlorphenyl)-oxiran als Fungizide zu verwenden (DE-A1-3218130.2, EP-A2-0196038, DE-A1-3218129). Ihre Wirkungen sind jedoch ungenügend.

Es wurde nun gefunden, daß Azolylmethylderivate der allgemeinen Formel I

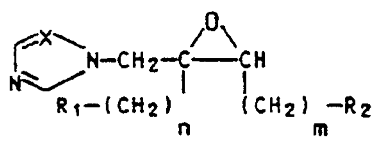

I,

in welcher $R_1$ und $R_2$ $C_1$-$C_5$-Alkyl, Naphthyl, Biphenyl, Dioxanyl oder Phenyl bedeuten, wobei diese Reste gegebenenfalls durch Halogen, Nitro, Phenoxy, Alkyl, Alkoxy, Amino oder Halogenalkyl mit jeweils 1 bis 4 Kohlenstoffatomen substituiert sind,

m und n eine ganze Zahl von 1 bis 5 oder 0 bedeuten mit der Maßgabe, daß die Summe m + n den Wert 1 oder mehr als 1 hat,

X CH oder N bedeutet, oder deren für Pflanzen verträgliche Säureadditionssalze oder Metallkomplexe eine bessere fungizide Wirkung, insbesondere gegen Getreidekrankheiten, haben als bekannte Azolverbindungen.

Die Verbindungen der Formel I enthalten chirale Zentren und werden im allgemeinen in Form von Racematen bzw. als Diastereomerengemische von erythro- bzw. threo-Formen erhalten. Die erythro- bzw. threo-Diastereomeren lassen sich bei den erfindungsgemäßen Verbindungen in üblicher Weise beispielsweise aufgrund ihrer unterschiedlichen löslichkeit oder durch Säulenchromatographie trennen und in reiner Form isolieren. Aus einem solchen isolierten Diastereomeren kann man mit bekannten Methoden einheitliche Enantiomere erhalten. Als fungizide Mittel kann man sowohl die einheitlichen Diastereomere bzw. Enantiomere wie auch deren bei der Synthese anfallende Gemische verwenden. Alle diese Verbindungen werden von der vorliegenden Erfindung umfaßt.

$R_1$ bzw. $R_2$ bedeuten beispielsweise : Methyl, Ethyl, Isopropyl, n-Propyl, n-Butyl, sec-Butyl, tert.-Butyl, n-Pentyl, Neopentyl, 1-Naphthyl, 2-Naphthyl, p-Biphenyl, Phenyl, Halogenphenyl, 2-Chlorphenyl, 2-Fluorphenyl, 2-Bromphenyl, 3-Chlorphenyl, 3-Bromphenyl, 3-Fluorphenyl, 4-Fluorphenyl, 4-Chlorphenyl, 4-Bromphenyl, 2,4-Dichlorphenyl, 2,3-Dichlorphenyl, 2,5-Dichlorphenyl, 2,6-Dichlorphenyl, 2-Chlor-6-fluorphenyl, Alkoxyphenyl, 2-Methoxyphenyl, 3-Methoxyphenyl, 4-Methoxyphenyl, 2,4-Dimethoxyphenyl, Alkylphenyl, 4-Ethylphenyl, 4-Isopropylphenyl, 4-tert- Butylphenyl, 4-tert.-Butyloxyphenyl, 2-Chlor-4-fluorphenyl, 2-Chlor-6-methylphenyl, 3,4-Dimethoxyphenyl, 3-Phenoxyphenyl, 4-Phenoxyphenyl, 3-Nitrophenyl, 4-Nitrophenyl, 3-Aminophenyl, 4-Aminophenyl, 2-Aminophenyl, 2-Trifluormethylphenyl, 3-Trifluormethylphenyl, 4-Trifluormethylphenyl, 1,3-Dioxan-2-yl, 1,4-Dioxan-1-yl,

Säureadditionssalze sind beispielsweise die Hydrochloride, Bromide, Sulfate, Nitrate, Phosphate, Oxalate oder Dodecylbenzolsulfonate. Die Wirksamkeit der Salze geht auf das Kation zurück, so daß das Anion i.a. beliebig gewählt werden kann. Die erfindungsgemäßen Wirkstoffsalze werden hergestellt durch Umsetzung der Azolylmethyloxirane mit den Säuren.

Metallkomplexe der Wirkstoffe oder ihre Salze können mit Kupfer, Zink, Zinn, Mangan, Eisen, Kobalt oder Nickel gebildet werden, indem man die Azolylmethyloxirane mit entsprechenden Metallsalzen umsetzt z.B. mit Kupfersulfat, Zinkchlorid, Zinnchlorid, Mangansulfat, Eisenchlorid, Kobaltsulfat.

Die Verbindungen der Formel I können z.B. hergestellt werden, indem man

a) eine Verbindung der Formel II

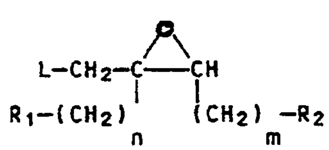

II,

in welcher $R^1$, $R^2$, D, m und n die oben angegebenen Bedeutungen haben und L eine nucleophil substituierbare Abgangsgruppe (OH, Halogen) darstellt, mit einer Verbindung der Formel III

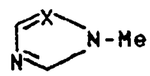 III,

in der Me ein Wasserstoffatom oder ein Metallatom (Na, K) bedeutet und X die oben angegebene Bedeutung hat, zur Umsetzung bringt, oder

b) eine Verbindung der Formel IV

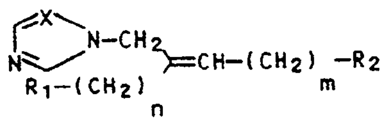 IV,

in welcher $R_1$, $R_2$, m, n und X die oben angegebene Bedeutung haben, in die Epoxide überführt.

Die Reaktion a) erfolgt — falls Me ein Wasserstoffatom bedeutet — gegebenenfalls in Gegenwart eines Lösungs- oder Verdünnungsmittels, gegebenenfalls unter Zusatz einer anorganischen oder organischen Base und gegebenenfalls unter Zusatz eines Reaktionsbeschleunigers bei Temperaturen zwischen 10 und 120°C. Zu den bevorzugten Lösungs- und Verdünnungsmitteln gehören Ketone wie Aceton, Methylethylketon oder Cyclohexanon, Nitrile wie Acetonitril oder Propionitril, Alkohole wie Methanol, Ethanol, iso-Propanol, n-Butanol oder Glycol, Ester wie Essigsäureethylester, Essigsäuremethylester oder Essigsäurebutylester, Ether wie Tetrahydrofuran, Diethylether, Dimethoxyethan, Dioxan oder Diisopropylether, Amide wie Dimethylformamid, Dimethylacetamid oder N-Methylpyrrolidon, ferner Dimethylsulfoxid, Sulfolan oder entsprechende Gemische.

Geeignete Basen, die gegebenenfalls auch als säurebindende Mittel bei der Reaktion verwendet werden können, sind beispielsweise Alkalihydroxid wie Lithium-, Natrium- oder Kaliumhydroxid, Alkalicarbonate wie Natrium-, Kalium- oder Caesiumcarbonat oder Natrium-, Kalium- oder Caesiumhydrogencarbonat, Pyridin oder 4-Dimethylaminopyridin. Es können aber auch andere übliche Basen verwendet werden.

Als Reaktionsbeschleuniger kommen vorzugsweise Metallhalogenide wie Natriumjodid oder Kaliumjodid, quartäre Ammoniumsalze wie Tetrabutylammoniumchlorid, -bromid, -jodid oder -hydrogensulfat, Benzyltriethylammoniumchlorid oder -bromid oder Kronenether wie 12-Krone-4, 15-Krone-5, 18-Krone-6, Dibenzo-18-Krone-6 oder Dicyclohexano-18-Krone-6 in Frage.

Die Umsetzung wird im allgemeinen bei Temperaturen zwischen 20 und 150°C, drucklos oder unter Druck, kontinuierlich oder diskontinuierlich durchgeführt.

Ist Me ein Metallatom (Na, K) wird die Reaktion a) gegebenenfalls in Gegenwart eines Lösungs- oder Verdünnungsmittels und gegebenenfalls unter Zusatz einer starken anorganischen oder organischen Base bei Temperaturen zwischen −10 und 120°C durchgeführt. Zu den bevorzugten Lösungs- und Verdünnungsmitteln gehören Amide wie Dimethylformamid, Diethylformamid, Dimethylacetamid, Diethylacetamid, N-Methylpyrrolidon, Hexamethyl-phosphorsäure-triamid, Sulfoxide wie Dimethylsulfoxid und Sulfolan.

Geeignete Basen, die gegebenenfalls auch als säurebindende Mittel bei der Reaktion verwendet werden können, sind beispielsweise Alkalihydride wie Lithium-, Natrium- und Kaliumhydrid, Alkaliamide wie Natrium- und Kaliumamid, ferner Natrium- oder Kalium-tert.-butoxid, Lithium-, Natrium- oder Kalium-triphenylmethyl und Naphthalinlithium, -natrium oder -kalium.

Für die Reaktion b) kommen als Verdünnungsmittel polare organische Lösungsmittel wie Nitrile, z.B. Acetonitril, Sulfoxide, z.B. Dimethylsulfoxid, Formamide, z.B. Dimethylformamid, Ketone,z.B. Aceton, Ether, z.B. Diethylether, Tetrahydrofuran und insbesondere Chlorkohlenwasserstoffe, z.B. Methylenchlorid und Chloroform, in Frage.

Man arbeitet im allgemeinen zwischen 0 und 100°C, vorzugsweise bei 20 bis 80°C. Bei Anwesenheit eines Lösungmittels wird zweckmäßigerweise beim Siedepunkt des jeweiligen Lösungsmittels gearbeitet.

Die neuen Ausgangsverbindungen II erhält man durch Epoxidierung der entsprechenden Olefine V :

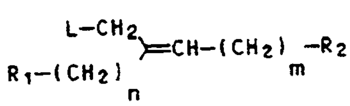 V

(vgl. G. Dittus in Houben-Weyl-Müller, Methoden der Organischen Chemie, Georg Thieme Verlag, Stuttgart, 1965, Bd. VI, 3, Seite 385 ff.)

Die Verbindung V stellt man her, indem man Olefine der Formel VI

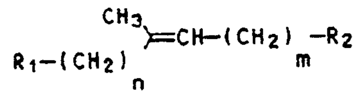 VI

nach bekannten Methoden in Allylposition halogeniert oder oxidiert.

Geeignete Halogenierungsreagenzien sind N-Chlor- und N-Bromsuccinimid in halogenierten Kohlenwasserstoffen wie Tetrachlorkohlenstoff, Trichlorethan oder Methylenchlorid bei Temperaturen zwischen 20 und 100°C. Zur Allyloxidation verwendet man Perester wie Perbenzoesäure-tert.-butylester oder Peressigsäure-tert.-butylester in Anwesenheit eines Schwermetallsalzes wie z.B. Kupfer-I-chlorid oder Kupfer-I-bromid. Man arbeitet in inerten Lösungsmitteln bei Temperaturen zwischen 10 und 100°C.

Die so erhaltenen Allylhalogenide bzw. -alkohole V werden anschließend in die entsprechenden Epoxide II (L = Halogen, OH) übergeführt. Dazu oxidiert man die Olefine V mit Peroxycarbonsäuren wie Perbenzoesäure, 3-Chlorperbenzoesäure, 4-Nitroperbenzoesäure, Monoperphthalsäure, Peressigsäure, Perpropionsäure, Permaleinsäure, Monoperbernsteinsäure, Perpelargonsäure oder Trifluorperessigsäure in indifferenten Lösungsmitteln, vorzugsweise chlorierten Kohlenwasserstoffen, z.B. Methylenchlorid, Chloroform, Tetrachlorkohlenstoff Dichlorethan, aber gegebenenfalls auch in Essigsäure, Essigester, Aceton oder Dimethylformamid, gegebenenfalls in Gegenwart eines Puffers wie Natriumacetat, Natriumcarbonat, Dinatriumhydrogenphosphat, Triton B. Man arbeitet zwischen 10 und 100°C und katalysiert die Reaktion gegebenenfalls z.B. mit Jod, Natriumwolframat oder Licht. Zur Oxidation eignen sich auch alkalische Lösungen von Wasserstoffperoxid (ca. 30%ig) in Methanol, Ethanol, Aceton oder Acetonitril bei 25 bis 30°C sowie Alkylhydroperoxide, z.B. tert.-Butylhydroperoxid, unter Zusatz eines Katalysators, z.B. Natriumwolframat, Perwolframsäure, Molybdänhexacarbonyl oder Vanadylacetylacetonat. Die genannten Oxidationsmittel lassen sich z.T. in situ erzeugen.

Während die so erhaltenen Epoxihalogenide II (L = Halogen) gemäß Verfahren a) sofort umgesetzt werden können, überführt man die entsprechenden Epoxidalkohole II (L = OH) in reaktive Ester, die dann mit den Verbindungen III gemäß Verfahren a) umgesetzt werden.

Die Darstellung der reaktiven Ester, die mit III umgesetzt werden, erfolgt nach allgemein bekannten Methoden (Houben-Weyl-Müller, Methoden der organischen Chemie, Georg Thieme Verlag, Stuttgart, 1955, Band 9, Seiten 388, 663, 671). Solche Ester sind beispielsweise Methansulfonsäureester, Trifluormethansulfonsäureester, 2,2,2-Trifluorethansulfonsäureester, Nonafluorbutansulfonsäureester, 4-Methylbenzolsulfonsäureester, 4-Brombenzolsulfonsäureester, 4-Nitrobenzolsulfonsäureester oder Benzolsulfonsäureester.

Die Verbindungen V lassen sich entsprechend allgemein bekannten Verfahren zur Olefinsynthese (Houben-Weyl-Müller, Methoden der organischen Chemie, Georg Thieme Verlag, Stuttgart, 1972 Bd. V, 1b) herstellen.

Die Verbindungen der Formel IV erhält man z.B. durch Umsetzung einer Verbindung der Formel VI mit einer Verbindung der Formel III.

Die folgenden Beispiele erläutern die Herstellung der Wirkstoffe.

I. Herstellung der Ausgangsstoffe

Vorschrift A

Zu einer Lösung von 35 g 2-Chlorbenzaldehyd in 300 ml Methanol werden 4,2 g Natriumhydroxid in 30 ml Wasser gegeben. Das Reaktionsgemisch wird auf 10°C gekühlt und schnell 36 g 2-(2-Formylethyl)-1,3-dioxan zugesetzt, wobei die Temperatur in der Lösung auf 30-40°C ansteigt. Nach 10 stündigem Rühren bei 40°C wird der farblosen Reaktionslösung 200 ml Wasser zugesetzt und die nun entstandene Emulsion mit Methyl-tert.-butylether ausgeschüttelt. Die organische Phase wird abgetrennt, über Natriumsulfat getrocknet und eingeengt. Nach Säulenfiltration des verbleibenden Rückstandes an Kieselgel (Essigester/n-Hexan = 1 : 9) erhält man 52 g (78%) E/Z-2-(-1,3-dioxan-2-yl-methyl)-3-(2-chlorphenyl)-propenal.

Vorschrift B

52 g E/Z-2-(-1,3-dioxan-2-yl-methyl)-3-(2-chlorphenyl)-propenal werden in 300 ml Methanol gelöst und 2,21 ml Natronlauge (konz.) zugesetzt. Die Reaktionslösung wird bei 0°C gerührt, während 14,3 g Wasserstoffperoxid (ca. 50 gew.%ig) langsam zugetropft werden, wobei die Innentemperatur von 30°C nicht überschritten wird. Nach beendeter Zugabe wird 6 Stunden bei Raumtemperatur (20°C) gerührt und anschließend 2,35 g Natriumborhydrid zugegeben, das in wenig 10% iger Natronlauge gelöst ist. Nachdem das Reaktiongemisch 18 Stunden bei Raumtemperatur rührte, wird der Lösung 200 ml Wasser zugesetzt und die entstandene Emulsion mit Methylenchlorid ausgeschüttelt. Die isolierte organische Phase wird daraufhin über Natriumsulfat getrocknet, eingeengt und der verbleibende Rückstand aus Isopropanol umkristallisiert. Man erhält 49 g (88%) cis-2-Hydroxymethyl-2-(1,3-dioxan-2-yl-methyl)-3-(2-chlorphenyl)-oxiran, Fp. : 79°C.

Vorschrift C

Zu einer Lösung von 49 g cis-2-(1,3-dioxan-2-yl-methyl)-3-(2-chlorphenyl)-oxiran in 200 ml Dichlormethan und 53 g Triethylamin werden bei Raumtemperatur 37,5 g 4-Methylbenzolsulfonsäurechlorid zugesetzt. Nach 24 Stunden wird das Reaktionsgemisch mit wässriger Natriumhydrogencarbonat-Lösung und Wasser gewaschen, über Natriumsulfat getrocknet und im Vakuum eingedampft. Aus dem Rückstand erhält man 59,5 g (80%) cis-2-(4-Methylphenylsulfonyloxymethyl)-2-(1,3-dioxan-2-yl-methyl)-3-(2-chlorphenyl)-oxiran, das anschließend mit Triazol gemäß Beispiel 1 weiter verarbeitet wird.

Vorschrift D

Zu einer Lösung von 0,6 g Natriumhydroxid und 16,9 g 4-Chlorbenzaldehyd in 100 ml Methanol werden bei 30°C 13,7 g 4,4-Dimethylpentanal innerhalb von drei Stunden zugetropft. Die Reaktionslösung wird bei 30°C für 30 Minuten gerührt, dann mit 10%iger Schwefelsäure auf pH 7 eingestelt, ausgefallendes Natriumsulfat abfiltriert und das Filtrat eingeengt. Bei der anschließenden Destillation des verbleibenden Rückstandes werden bei 0,4 mbar und 117°C Übergangstemperatur 21,6 g (76%) Z-2-(2,2-Dimethyl-propyl)-3-(4-chlorphenyl)-propenal erhalten.

Vorschrift E

Zu einer Lösung von 47,3 g Z-2-(2,2-Dimethyl-propyl)-3-(4-chlorphenyl)-propenal wird 2,1 g Natriumborhydrid gegeben, das in wenig 10%iger Natronlauge gelöst ist. Nachdem das Reaktionsgemisch 18 Stunden bei Raumtemperatur rührte, wird der Lösung 200 ml Wasser zugesetzt und die entstandene Emulsion mit Methylenchlorid ausgeschüttelt. Die isolierte organische Phase wird daraufhin über Natriumsulfat getrocknet und eingeengt. Man erhält 45,8 g (96%) Z-1-(4-Chlorphenyl)-2-hydroxymethyl-4,4-dimethyl-pent-1-en.

Vorschrift F

Zu einer Lösung von 45,8 g Z-1-(4-Chlorphenyl)-2-hydroxymethyl-4,4-dimethyl-pent-1-en in 200 ml Methylenchlorid und 59 g Triethylamin werden bei Raumtemperatur 42 g 4-Methylbenzolsulfonsäurechlorid zugesetzt. Nach 24 Stunden wird das Reaktionsgemisch mit wäßriger Natriumhydrogencarbonat-Lösung und Wasser gewaschen, über Natriumsulfat getrocknet und im Vakuum eingedampft. Aus dem Rückstand erhält man 73,1 g (98%) Z-1-(4-Chlorphenyl)-2-(4-Methylphenylsulfonyloxymethyl)-4,4-dimethyl-pent-1-en.

II. Herstellung der Endprodukte

Beispiel 1

Eine Lösung von 9,4 g 1,2,4-Triazol in 100 ml N-Methylpyrrolidon wird mit 5,2 g Natriumhydroxid versetzt und für 30 Minuten auf 50°C erwärmt. Nachdem das Reaktionsgemisch auf Raumtemperatur gekühlt wurde, wird der Lösung 59,5 g cis-2-(4-Methylphenylsulfonyloxymethyl)-2-(1,3-dioxan-2-yl-methyl)-3-(2-chlorphenyl)-oxiran, das in 100 ml N-Methylpyrrolidon gelöst ist, langsam zugetropft und 12 Stunden bei Raumtemperatur gerührt. Anschließend wird 200 ml Wasser zugegeben und mehrmals mit Methyl-tert.-butylether extrahiert ; die organische Phase zweimal mit Wasser gewaschen, über Natriumsulfat getrocknet und eingeengt. Man erhält durch Kristallisation aus Methyl-tert.-butylether/n-Hexan 36,8 g (81%) cis-2-(1,2,4-Triazol-1-yl-methyl)-2-(1,3-dioxan-2-yl-methyl)-3-(2-chlorphenyl)-oxiran mit dem Schmelzpunkt 96-111°C (Verbindung Nr. 1).

Entsprechend Beispiel 1 können die in der Tabelle 1 aufgeführten Verbindungen hergestellt werden.

Beispiel 2

Eine Lösung von 16,8 g 1,2,4-Triazol in 150 ml N-Methylpyrrolidon wird mit 9,2 g Natriumhydroxid versetzt und für 30 Minuten auf 50°C erwärmt. Nachdem das Reaktionsgemisch auf Raumtemperatur gekühlt wurde, wird der Lösung 73,1 g Z-1-(4-Chlorphenyl)-2-(4-methylphenylsulfonyloxymethyl)-4,4-dimethyl-pent-1-en, das in 100 ml N-Methylpyrrolidon gelöst ist, langsam zugetropft und 12 Stunden bei Raumtemperatur gerührt.

Anschließend wird 200 ml Wasser zugegeben und mehrmals mit Methyl-tert.-butylether extrahiert, die organische Phase zweimal mit Wasser gewaschen, über Natriuimsulfat getrocknet und eingeengt. Der verbleibende Rückstand wird einer Chromatographie an Kieselgel mit n-Hexan/Essigester = 9 : 1 unterworfen, wobei 38 g (71%) Z-1-(4-Chlorphenyl)-2-(1,2,4-triazol-1-yl-methyl)-4,4-dimethyl-pent-1-en erhalten werden, das aus Methyl-tert.-butylether/n-Hexan kristallisiert werden kann, Fp. : 81-84°C (Verbindung Nr. A1).

Entsprechend Beispiel 2 können die in der Tabelle 2 aufgeführten Verbindungen hergestellt werden.

Tabelle 1

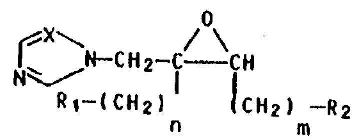 I,

| Bsp. | n | m | $R_1$ | $R_2$ | X | Schmp./IR |
|---|---|---|---|---|---|---|
| 1 | 1 | 0 | 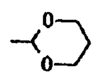 | 2-Cl-$C_6H_4$ | N | 96-111°C |
| 2 | 1 | 0 | tert.-butyl | 4-Cl-$C_6H_4$ | N | 59- 67°C |
| 3 | 1 | 0 | 4-F-$C_6H_4$ | tert.-butyl | N | - |
| 4 | 1 | 0 | 4-F-$C_6H_4$ | 2-Cl-$C_6H_4$ | N | Harz |
| 5 | 1 | 0 | tert.-butyl | Cyclohexyl | N | 2929, 1506, 1274, 1139, 1016 $cm^{-1}$ |
| 6 | 1 | 1 | tert.-butyl | 4-Cl-$C_4H_6$ | N | 70- 77°C |
| 7 | 1 | 1 | 4-F-$C_6H_4$ | tert.-butyl | N | 75- 77°C |
| 8 | 1 | 2 | 4-F-$C_6H_4$ | 4-F-$C_6H_4$ | N | Harz |
| 9 | 1 | 2 | 4-Cl-$C_6H_4$ | tert.-butyl | N | Harz |
| 10 | 0 | 1 | tert.-butyl | 2-Cl-$C_6H_4$ | N | - |
| 11 | 0 | 1 | tert.-butyl | 4-Cl-$C_6H_4$ | N | - |
| 12 | 0 | 1 | tert.-butyl | 4-F-$C_6H_4$ | N | 49- 52°C |
| 13 | 0 | 1 | 4-F-$C_6H_4$ | 2-Cl-$C_6H_4$ | N | - |
| 14 | 0 | 1 | 4-F-$C_6H_4$ | 2-Cl-$C_6H_4$ | CH | - |
| 15 | 0 | 1 | 4-F-$C_6H_4$ | 3-Cl-$C_6H_4$ | N | - |
| 16 | 0 | 1 | 4-F-$C_6H_4$ | 3-Cl-$C_6H_4$ | CH | - |
| 17 | 0 | 1 | 4-F-$C_6H_4$ | 4-Cl-$C_6H_4$ | N | - |
| 18 | 0 | 1 | 4-F-$C_6H_4$ | 4-Cl-$C_6H_4$ | CH | - |

| Bsp. | n | m | $R_1$ | $R_2$ | X | Schmp./IR |
|---|---|---|---|---|---|---|
| 19 | 0 | 1 | $4-F-C_6H_4$ | $2,4-Cl_2-C_6H_3$ | N | - |
| 20 | 0 | 1 | $4-F-C_6H_4$ | $2,4-Cl_2-C_6H_3$ | CH | - |
| 21 | 0 | 1 | $4-F-C_6H_4$ | $2-CF_3-C_6H_4$ | N | - |
| 22 | 0 | 1 | $4-F-C_6H_4$ | $2-CF_3-C_6H_4$ | CH | - |
| 23 | 0 | 1 | $4-F-C_6H_4$ | $4-CF_3-C_6H_4$ | CH | - |
| 24 | 0 | 1 | $4-F-C_6H_4$ | $4-CF_3-C_6H_4$ | N | - |
| 25 | 0 | 1 | $4-Cl-C_6H_4$ | $2-Cl-C_6H_4$ | N | - |
| 26 | 0 | 1 | $4-Cl-C_6H_4$ | $2-Cl-C_6H_4$ | CH | - |
| 27 | 0 | 1 | $4-Cl-C_6H_4$ | $4-Cl-C_6H_4$ | N | - |
| 28 | 0 | 1 | $4-Cl-C_6H_4$ | $4-Cl-C_6H_4$ | CH | - |
| 29 | 0 | 1 | $4-Cl-C_6H_4$ | $4-F-C_6H_4$ | N | - |
| 30 | 0 | 1 | $4-Cl-C_6H_4$ | $4-F-C_6H_4$ | CH | - |
| 31 | 0 | 1 | $4-Cl-C_6H_4$ | $2-F-C_6H_4$ | N | - |
| 32 | 0 | 1 | $4-Cl-C_6H_4$ | $2,4-Cl_2-C_6H_3$ | N | - |
| 33 | 0 | 1 | $4-Cl-C_6H_4$ | $2,4-Cl_2-C_6H_3$ | CH | - |
| 34 | 0 | 1 | $2,4-Cl_2-C_6H_3$ | $2-Cl-C_6H_4$ | N | - |
| 35 | 0 | 1 | $2,4-Cl_2-C_6H_3$ | $2-Cl-C_6H_4$ | N | - |
| 36 | 0 | 1 | $2,4-Cl_2-C_6H_3$ | $4-F-C_6H_4$ | N | - |
| 37 | 0 | 1 | $2,4-Cl_2-C_6H_3$ | $4-F-C_6H_4$ | CH | - |
| 38 | 0 | 1 | $2,4-Cl_2-C_6H_3$ | $2,4-Cl_2-C_6H_3$ | N | - |
| 39 | 0 | 2 | $4-F-C_6H_4$ | $4-Cl-C_6H_4$ | N | - |
| 40 | 0 | 2 | $4-F-C_6H_4$ | $3-Cl-C_6H_4$ | N | - |
| 41 | 0 | 2 | $4-F-C_6H_4$ | $2-Cl-C_6H_4$ | N | - |

| Bsp. | n | m | $R_1$ | $R_2$ | X | Schmp./IR |
|---|---|---|---|---|---|---|
| 42 | 0 | 2 | 4-Cl-$C_6H_4$ | 4-Cl-$C_6H_4$ | N | - |
| 43 | 0 | 2 | 4-Cl-$C_6H_4$ | 2-Cl-$C_6H_4$ | N | - |
| 44 | 0 | 2 | 4-Cl-$C_6H_4$ | 4-F-$C_6H_4$ | N | - |
| 45 | 0 | 2 | 4-Cl-$C_6H_4$ | 2-F-$C_6H_4$ | N | - |
| 46 | 0 | 2 | 4-Cl-$C_6H_4$ | 2-$OCH_3$-$C_6H_4$ | N | - |
| 47 | 0 | 2 | 4-$OCH_3$-$C_6H_4$ | 4-Cl-$C_6H_4$ | N | - |
| 48 | 0 | 2 | 4-$OCH_3$-$C_6H_4$ | 2-F-$C_6H_4$ | N | - |
| 49 | 0 | 2 | tert.-butyl | Cyclohexyl | N | - |
| 50 | 0 | 2 | 4-Cl-$C_6H_4$ | Cyclohexyl | N | - |
| 51 | 0 | 2 | 4-Cl-$C_6H_4$ | Cyclohexyl | N | - |
| 52 | 0 | 2 | 4-F-$C_6H_4$ | Cyclopentyl | N | - |
| 53 | 2 | 2 | 2-Cl-$C_6H_4$ | 2-Cl-$Cl_6H_4$ | N | - |
| 54 | 2 | 2 | 4-Cl-$C_6H_4$ | Cyclohexyl | N | - |
| 55 | 1 | 3 | 4-F-$C_6H_4$ | 4-F-$C_6H_4$ | N | - |
| 56 | 1 | 4 | 4-F-$C_6H_4$ | 2-F-$C_6H_4$ | N | - |
| 57 | 2 | 4 | 2,4-$Cl_2$-$C_6H_3$ | 2-Cl-$C_6H_4$ | N | - |
| 58 | 3 | 3 | 2-Cl-$C_6H_4$ | 3-Cl-$C_6H_4$ | N | - |
| 59 | 0 | 5 | 2-F-$C_6H_4$ | 4-$CH_3$-$C_6H_4$ | N | - |
| 60 | 2 | 5 | 4-F-$C_6H_4$ | 4-F-$C_6H_4$ | N | - |
| 61 | 1 | 0 | $C_6H_5$ | 2-Cl-$C_6H_4$ | N | - |
| 62 | 0 | 1 | 2-Cl-$C_6H_4$ | $C_6H_5$ | N | - |

Tabelle 2

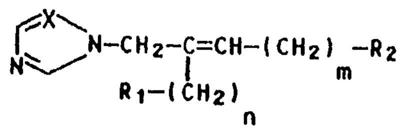

| Bsp. | n | m | $R_1$ | $R_2$ | X | Schmp./IR | Isomer |
|---|---|---|---|---|---|---|---|
| A 1 | 1 | 0 | tert.-butyl | $4-Cl-C_6H_4$ | N | 81 - 84$^0$C | cis |
| A 2 | 1 | 0 | tert.-butyl | $4-Cl-C_6H_4$ | CH | - | - |
| A 3 | 1 | 0 | tert.-butyl | $4-F-C_6H_4$ | N | - | - |
| A 4 | 1 | 0 | tert.-butyl | Cyclohexyl | N | - | - |
| A 5 | 1 | 0 | tert.-butyl | Cyclohexyl | CH | - | - |
| A 6 | 1 | 0 | $4-F-C_6H_4$ | tert.-butyl | N | - | - |
| A 7 | 1 | 0 | $4-F-C_6H_4$ | tert.-butyl | CH | - | - |
| A 8 | 1 | 0 | $4-Cl-C_6H_4$ | tert.-butyl | N | - | - |
| A 9 | 1 | 1 | $4-F-C_6H_4$ | tert.-butyl | N | - | - |
| A10 | 1 | 1 | $4-F-C_6H_4$ | tert.-butyl | CH | - | - |
| A11 | 1 | 2 | $4-F-C_6H_4$ | $4-F-C_6H_4$ | N | - | - |
| A12 | 1 | 2 | $4-F-C_6H_4$ | $4-F-C_6H_4$ | CH | - | - |
| A13 | 1 | 2 | $4-Cl-C_6H_4$ | tert.-butyl | N | Harz | E/Z = 55:45 |
| A14 | 0 | 1 | tert.-butyl | $2-Cl-C_6H_4$ | N | - | - |
| A15 | 0 | 1 | tert.-butyl | $2-Cl-C_6H_4$ | CH | - | - |
| A16 | 0 | 1 | $4-F-C_6H_4$ | $2-Cl-C_6H_4$ | N | - | - |
| A17 | 0 | 1 | $4-F-C_6H_4$ | $2-Cl-C_6H_4$ | CH | - | - |
| A18 | 0 | 1 | $4-Cl-C_6H_4$ | $2-Cl-C_6H_4$ | N | - | - |

| Bsp. | n | m | $R_1$ | $R_2$ | X | Schmp./IR | Isomer |
|---|---|---|---|---|---|---|---|
| A19 | 0 | 1 | $4-Cl-C_6H_4$ | $2-Cl-C_6H_4$ | CH | - | - |
| A20 | 0 | 1 | $2,4-Cl_2-C_6H_3$ | $2-Cl-C_6H_4$ | N | - | - |
| A21 | 0 | 1 | $2,4-Cl_2-C_6H_3$ | $2-Cl-C_6H_4$ | CH | - | - |
| A22 | 0 | 1 | $2,4-Cl_2-C_6H_3$ | $4-F-C_6H_4$ | N | - | - |
| A23 | 0 | 2 | $4-F-C_6H_4$ | $4-Cl-C_6H_4$ | N | - | - |
| A24 | 0 | 2 | $4-F-C_6H_4$ | $4-Cl-C_6H_4$ | CH | - | - |
| A25 | 0 | 2 | $4-F-C_6H_4$ | $2-Cl-C_6H_4$ | N | - | - |
| A26 | 0 | 2 | $4-OCH_3-C_6H_4$ | $4-Cl-C_6H_4$ | N | - | - |
| A27 | 0 | 2 | $4-OCH_3-C_6H_4$ | $4-Cl-C_6H_4$ | CH | - | - |
| A28 | 0 | 2 | tert.-butyl | Cyclohexyl | N | - | - |
| A29 | 0 | 2 | $4-Cl-C_6H_4$ | Cyclohexyl | N | - | - |
| A30 | 0 | 2 | $4-Cl-C_6H_4$ | Cyclohexyl | CH | - | - |
| A31 | 0 | 2 | $4-F-C_6H_4$ | Cyclohexyl | N | - | - |
| A32 | 0 | 1 | $C_6H_5$ | $2-Cl-C_6H_4$ | N | - | - |
| A33 | 0 | 1 | $C_6H_5$ | $2-Cl-C_6H_4$ | CH | - | - |
| A34 | 1 | 0 | $2-Cl-C_6H_4$ | $C_6H_5$ | N | - | - |
| A35 | 1 | 0 | $2-Cl-C_6H_4$ | $C_6H_5$ | CH | - | - |
| A36 | 2 | 2 | $2-Cl-C_6H_4$ | $2-Cl-C_6H_4$ | N | - | - |
| A37 | 2 | 2 | $2-Cl-C_6H_4$ | $2-Cl-C_6H_4$ | CH | - | - |
| A38 | 1 | 3 | $4-F-C_6H_4$ | $4-F-C_6H_4$ | N | - | - |
| A39 | 1 | 3 | $4-F-C_6H_4$ | $4-F-C_6H_4$ | CH | - | - |
| A40 | 1 | 4 | $4-F-C_6H_4$ | $2-F-C_6H_4$ | N | - | - |
| A41 | 1 | 4 | $4-F-C_6H_4$ | $2-F-C_6H_4$ | CH | - | - |

| Bsp. | n | m | $R_1$ | $R_2$ | X | Schmp./IR | Isomer |
|---|---|---|---|---|---|---|---|
| A42 | 2 | 4 | $2,4-Cl_2-C_6H_3$ | $2-Cl-C_6H_4$ | N | - | - |
| A43 | 3 | 3 | $2-Cl-C_6H_4$ | $3-Cl-C_6H_4$ | N | - | - |
| A44 | 3 | 3 | $2-Cl-C_6H_4$ | $3-Cl-C_6H_4$ | CH | - | - |
| A45 | 0 | 5 | $2-F-C_6H_4$ | $4-CH_3-C_6H_4$ | N | - | - |
| A46 | 0 | 5 | $2-F-C_6H_4$ | $4-CH_3-C_6H_4$ | CH | - | - |
| A47 | 2 | 5 | $4-F-C_6H_4$ | $4-F-C_6H_4$ | N | - | - |
| A48 | 2 | 5 | $4-F-C_6H_4$ | $4-F-C_6H_4$ | CH | - | - |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis, Mais, Rasen, Baumwolle, Soja, Kaffee, Zuckerrohr, Obst und Zierpflanzen im Gartenbau, Weinbau sowie Gemüse — wie Gurken, Bohnen und Kürbisgewächse —.

Die neuen Verbindungen sind insbesondere geeignet zur Bekämpfung folgender Pflanzenkrankheiten :

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara Viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken ; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage : Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser ; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind :

I. Man vermischt 90 Gew.-Teile der Verbindung Nr. 2 mit 10 Gew.-Teilen N-Methyl-α-pyrrolidon und erhält eine Lösung, die zur Anwendung in Form kleinster Tropfen geeignet ist.

II. 20 Gew.-Teile der Verbindung Nr. 6 werden in einer Mischung gelöst, die aus 80 Gew.-Teilen Xylol, 10 Gew.-Teilen des Anlagerungsproduktes von 8 bis 10 Mol Ethylenoxid an 1 Mol Ölsäure-N-monoethanolamid, 5 Gew.-Teilen Calciumsalz der Dodecylbenzolsulfonsäure und 5 Gew.-Teilen

des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 7 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 8 werden in einer Mischung gelöst, die aus 25 Gew.-Teilen Cyclohexanol, 65 Gew.-Teilen einer Mineralölfraktion vom Siedepunkt 210 bis 280°C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 4 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 5 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 7 werden mit 10 Gew.-Teilen Natriumsalz eines Phenolsulfonsäure-harnstoff-formaldehyd-Kondensates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 8 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde cis-2-(1,2,4-Triazol-1-ylmethyl)-2-(tert.-butyl)-3-(4-chlorphenyll-oxiran (A)—bekannt aus DE 3218130.2 -benutzt.

Anwendungsbeispiel 1

Wirksamkeit gegen Weizenbraunrost

Blätter von in Töpfen gewachsenen Weizensämlingen der Sorte "Frühgold" wurden mit Sporen des Braunrostes (Puccinia recondita) bestäubt. Danach wurden die Töpfe für 24 Stunden bei 20 bis 22°C in eine Kammer mit hoher luftfeuchtigkeit (90 bis 95%) gestellt. Während dieser Zeit keimten die Sporen aus und die Keimschläuche drangen in das Blattgewebe ein. Die infizierten Pflanzen wurden anschließend mit wäßrigen Spritzbrühen, die 80% Wirkstoff und 20% Emulgiermittel in der Trockensubstanz enthielten, tropfnaß gespritzt. Nach dem Abtrocknen des Spritzbelages wurden die Versuchspflanzen im Gewächshaus bei Temperaturen zwischen 20 und 22°C und 65 bis 70% relativer luftfeuchte aufgestellt. Nach 8 Tagen wurde daß Ausmaß der Rostpilzentwicklung auf den Blättern ermittelt.

Das Ergebnis zeigt, daß die Wirkstoffe 2, 6, 7 und 8 bei der Anwendung als 0,025%ige (Gew.%) Spritzbrühen eine bessere fungizide Wirkung zeigen (97%) als der bekannte Vergleichswirkstoff A (60%).

Anwendungsbeispiel 2

Wirksamkeit gegen Pyrenophora teres

Gerstenkeimlinge der Sorte "Igri" wurden im Zweiblattstadium mit wäßrigen Suspensionen, die 80% Wirkstoff und 20% Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18°C gestellt. Anschließend wurden die Pflanzen im Gewächshaus bei 20-22°C und 70% relativer Luftfeuchtigkeit für weitere 5 Tage kultiviert. Dann wurde das Ausmaß der Symptomentwicklung ermittelt.

Das Ergebnis zeigt, daß die Wirkstoffe 2, 4, 5, 7 und 8 bei der Anwendung als 0,05%ige Spritzbrühe eine sehr gute fungizide Wirkung (90%) zeigen.

## Patentansprüche

1. Azolylmethylderivate der allgemeinen Formel I

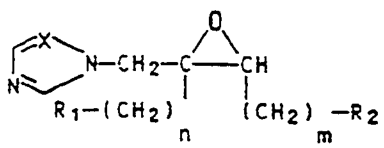

I,

in welcher $R_1$ und $R_2$ $C_1$-$C_5$-Alkyl, Naphthyl, Biphenyl, Dioxanyl oder Phenyl bedeuten, wobei diese Reste gegebenenfalls durch Halogen, Nitro, Phenoxy, Alkyl, Alkoxy, Amino oder Halogenalkyl mit jeweils 1 bis 4 Kohlenstoffatomen substituiert sind,
m und n eine ganze Zahl von 1 bis 5 oder 0 bedeuten mit der Maßgabe, daß die Summe m + n den Wert 1 oder mehr als 1 hat,
X CH oder N bedeutet, sowie deren für Pflanzen verträgliche Säureadditionssalze oder Metallkomplexe.

2. Verfahren zur Herstellung der Azolylmethylderivate der Formel I

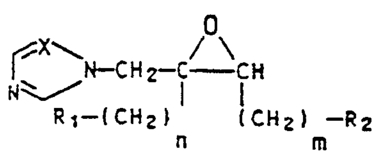

I,

in welcher $R_1$ und $R_2$ $C_1$-$C_5$-Alkyl, Naphthyl, Biphenyl, Dioxanyl oder Phenyl bedeuten, wobei diese Reste gegebenenfalls durch Halogen, Nitro, Phenoxy, Alkyl, Alkoxy, Amino oder Halogenalkyl mit jeweils 1 bis 4 Kohlenstoffatomen substituiert sind,
m und n eine ganze Zahl von 1 bis 5 oder 0 bedeuten mit der Maßgabe, daß die Summe m + n den Wert 1 oder mehr als 1 hat,
X CH oder N bedeutet, dadurch gekennzeichnet, daß man

a) eine Verbindung der Formel II

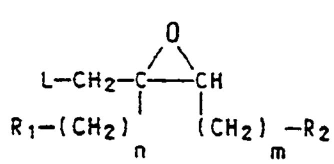

II,

in welcher $R_1$, $R_2$, m und n die oben angegebenen Bedeutungen haben und L eine nucleophil substituierbare Abgangsgruppe darstellt, mit einer Verbindung der Formel III

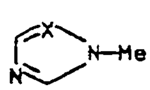

III,

in der Me ein Wasserstoffatom oder ein Metallatom bedeutet und X die oben angegebene Bedeutung hat, zur Umsetzung bringt, oder

b) eine Verbindung der Formel IV

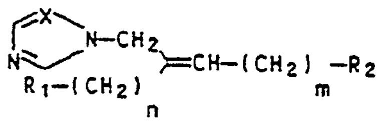

IV,

in welcher $R_1$, $R_2$, m, n und X die oben angegebene Bedeutung haben, in das Oxiran überführt und die so erhaltenen Verbindungen gegebenenfalls in ihre Salze mit für Pflanzen verträglichen Säuren überführt.

3. Fungizides Mittel, enthaltend einen Trägerstoff und ein Azolylmethylderivat der allgemeinen Formel I

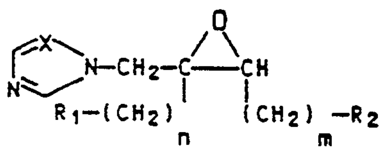

I,

in welcher $R_1$ und $R_2$ $C_1$-$C_5$-Alkyl, Haphthyl, Biphenyl, Dioxanyl oder Phenyl bedeuten, wobei diese Reste gegebenenfalls durch Halogen, Hitro, Phenoxy, Alkyl, Alkoxy, Amino oder Halogenalkyl mit jeweils 1 bis 4 Kohlenstoffatomen substituiert sind,
m und n eine ganze Zahl von 1 bis 5 oder 0 bedeuten mit der Maßgabe, daß die Summe m + n den Wert 1 oder mehr als 1 hat,
X CH oder N bedeutet, oder dessen für Pflanzen verträgliches Säureadditionssalz oder Metallkomplex.

4. verfahren zur Bekämpfung von Pilzen, dadurch gekennzeichnet, daß man eine fungizid wirksame Menge eines Azolylmethylderivats der allgemeinen Formel I

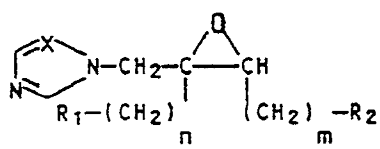

I,

in welcher $R_1$ und $R_2$ $C_1$-$C_5$-Alkyl, Naphthyl, Biphenyl, Dioxanyl oder Phenyl bedeuten, wobei diese Reste gegebenenfalls durch Halogen, Nitro, Phenoxy, Alkyl, Alkoxy, Amino oder Halogenalkyl mit jeweils 1 bis 4 Kohlenstoffatomen substituiert sind,
m und n eine ganze Zahl von 1 bis 5 oder 0 bedeuten mit der Maßgabe, daß die Summe m + n den Wert 1 oder mehr als 1 hat,
X CH oder N bedeutet, oder dessen für Pflanzen verträgliches Säureadditionssalz oder Metallkomplex auf die Pilze oder auf die durch Pilzbefall bedrohte Materialien, Flächen, Pflanzen oder Saatgüter einwirken läßt.

5. Verbindung gemäß Anspruch 1, dadurch gekennzeichnet, daß $R^1$ tert.-Butyl, $R^2$ (-Chlorphenyl, n den Wert 1, m den Wert 0 und X N bedeutet.

6. Verbindung gemäß Anspruch 1, dadurch gekennzeichnet, daß $R^1$ 4-Fluorphenyl, $R^2$ tert.-Butyl, n den Wert 1, m den Wert 1 und X N bedeutet.

7. Verbindung gemäß Anspruch 1, dadurch gekennzeichnet, daß $R^1$ tert.-Butyl, $R^2$ 4-Chlorphenyl, n den Wert 1, m den Wert 1 und X N bedeutet.

8. Verbindung der Formel

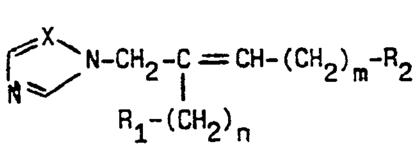

in welcher $R_1$ und $R_2$ $C_1$-$C_5$-Alkyl, Naphthyl, Biphenyl, Dioxanyl oder Phenyl bedeuten, wobei diese Reste gegebenenfalls durch Halogen, Nitro, Phenoxy, Alkyl, Alkoxy, Amino oder Halogenalkyl mit jeweils 1 bis 4 Kohlenstoffatomen substituiert sind,
m und n eine ganze Zahl von 1 bis 5 oder 0 bedeuten mit der Maßgabe, daß die Summe m + n den Wert 1 oder mehr als 1 hat,
X CH oder N bedeutet, sowie deren für Pflanzen verträgliche Säureadditionssalze.

9. Verbindung gemäß Anspruch 8, dadurch gekennzeichnet, daß $R^1$ tert-Butyl, $R^2$ 4-Chlorphenyl, n den Wert 1, m den Wert 0 und X N bedeutet, wobei die Verbindung als Z-Isomeres vorliegt.

## Claims

1. An azolylmethyl derivative of the general formula I

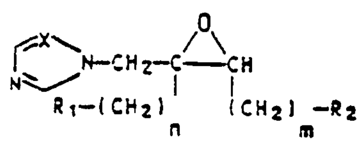
I.

where $R_1$ and $R_2$ are $C_1$-$C_5$-alkyl, naphthyl, biphenyl, dioxanyl or phenyl, each of these radicals being unsubstituted or substituted by halogen, nitro, phenoxy, alkyl, alkoxy, amino or haloalkyl, each having 1 to 4 carbon atoms,
m and n are each an integer from 1 to 5 or 0, with the proviso that the sum of m + n is 1 or more than 1,
X is CH or N, or a plant-tolerated acid addition salt thereof or a metal complex thereof.

2. A process for preparing an azolylmethyl derivative of the formula I

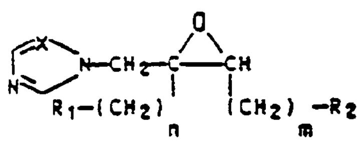
I.

where $R_1$ and $R_2$ are $C_1$-$C_5$-alkyl, naphthyl, biphenyl, dioxanyl or phenyl, each of these radicals being unsubstituted or substituted by halogen, nitro, phenoxy, alkyl, alkoxy, amino or haloalkyl, each having 1 to 4 carbon atoms,
m and n are each an integer from 1 to 5 or 0, with the proviso that the sum of m + n is 1 or more than 1, and X is CH or N, wherein

a) a compound of the formula II

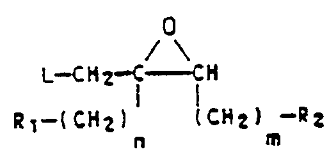
II.

where $R_1$, $R_2$, m and n have the abovementioned meanings and L is a nucleophilically substitutable leaving group, is reacted with a compound of the formula III

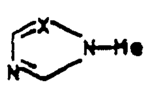
III.

where Me is hydrogen or a metal atom and X has the abovementioned meanings, or

b) a compound of the formula IV

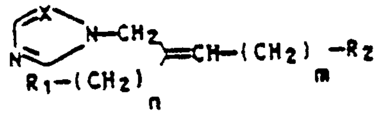

IV.

where $R_1$, $R_2$, m, n and X have the abovementioned meanings, is converted into the oxirane and the compound thus obtained is, if required, converted into its salts with plant-tolerated acids.

3. A fungicide containing a carrier and an azolylmethyl derivative of the general formula I

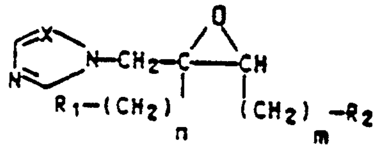

I.

where $R_1$ and $R_2$ are $C_1$-$C_5$-alkyl, naphthyl, biphenyl, dioxanyl or phenyl, each of these radicals being unsubstituted or substituted by halogen, nitro, phenoxy, alkyl, alkoxy, amino or haloalkyl, each having 1 to 4 carbon atoms,
m and n are each an integer from 1 to 5 or 0, with the proviso that the sum of m + n is 1 or more than 1, and X is CH or N, or a plant-tolerated acid addition salt or metal complex thereof.

4. A process for controlling fungi, wherein a fungicidal amount of an azolylmethyl derivative of the general formula I

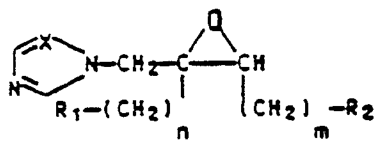

I.

where $R_1$ and $R_2$ are each $C_1$-$C_5$-alkyl, naphthyl, biphenyl, dioxanyl or phenyl, each of these radicals being unsubstituted or substituted by halogen, nitro, phenoxy, alkyl, alkoxy, amino or haloalkyl, each having 1 to 4 carbon atoms,
m and n are each an integer from 1 to 5 or 0, with the proviso that the sum of m + n is 1 or more than 1, and X is CH or N, or a plant-tolerated acid addition salt or metal complex thereof, is allowed to act on the fungi or on the materials, areas, plants or seeds threatened by fungal attack.

5. A compound as claimed in claim 1, where $R^1$ is tert-butyl, $R^2$ is 4-chlorophenyl, n is 1, m is 0 and X is N.

6. A compound as claimed in claim 1, where $R^1$ is 4-fluorophenyl, $R^2$ is tert-butyl, n is 1, m is 1 and X is N.

7. A compound as claimed in claim 1, where $R^1$ is tert-butyl, $R^2$ is 4-chlorophenyl, n is 1, m is 1 and X is N.

8. A compound of the formula

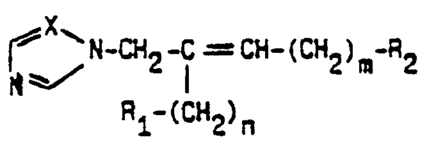

where $R_1$ and $R_2$ are $C_1$-$C_5$-alkyl, naphthyl, biphenyl, dioxanyl or phenyl, each of these radicals being unsubstituted or substituted by halogen, nitro, phenoxy, alkyl, alkoxy, amino or haloalkyl, each having 1 to 4 carbon atoms,
m and n are each an integer from 1 to 5 or 0, with the proviso that the sum of m + n is 1 or more than 1, and X is CH or N, or a plant-tolerated acid addition salt thereof.

9. A compound as claimed in claim 8, where $R^1$ is tert-butyl, $R^2$ is 4 -chlorophenyl, n is 1, m is 0 and X is N, the compound being a Z isomer.

**Revendications**

1. Dérivés azolylméthyliques de formule générale I

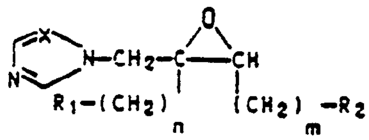

I.

dans laquelle R[1] et R[2] représentent des groupes alkyle en $C_1$-$C_5$, naphtyle, biphényle, dioxannyle ou phényle, ces groupes pouvant eux-mêmes être éventuellement substitués par des halogènes, des groupes nitro, phénoxy, alkyle, alcoxy, amino ou halogénoalkyle contenant chacun 1 à 4 atomes de carbone,
m et n sont des nombres entiers dont la valeur va de 1 à 5 ou sont égaux à 0, sous réserve que la somme m + n est égale à 1 ou a une valeur supérieure à 1,
X représente CH ou N, et leurs sels formés par addition avec des acides ou complexes métalliques tolérés par les végétaux.

2. Procédé de préparation des dérivés azolylméthyliques de formule I

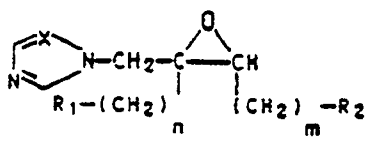

I.

dans laquelle $R_1$ et $R_2$ représentent des groupes alkyle en $C_1$-$C_5$, naphtyle, biphényle, dioxannyle ou phényle, ces groupes pouvant eux-mêmes être éventuellement substitués par des halogènes, des groupes nitro, phénoxy, alkyle, alcoxy, amino ou halogénoalkyle contenant chacun 1 à 4 atomes de carbone,
m et n sont des nombres entiers allant de 1 a 5 ou sont égaux à 0 sous réserve que la somme m + n est égale à 1 ou a une valeur supérieure à 1,
X représente CH ou N, caractérisé en ce que :

a) on fait réagir un composé de formule II

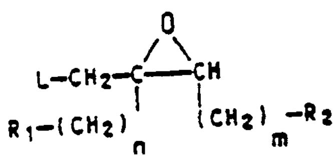

II.

dans laquelle $R_1$, $R_2$, m et n ont les significations indiquées ci-dessus, et L représente un groupe éliminable par substitution nucléophile, avec un composé de formule III

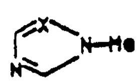

III.

dans laquelle Me représente un atome d'hydrogène ou un atome métallique et X a les significations indiquées cidessus, ou bien

b) on convertit un composé de formule IV

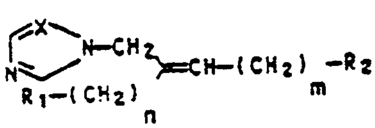

IV.

dans laquelle $R_1$, $R_2$, m, n et X ont les significations indiquées ci-dessus, en l'oxiranne et le cas échéant on convertit les composés ainsi obtenus en leurs sels d'acides tolérés par les végétaux.

3. Produit fongicide, contenant un véhicule et un dérivé azolylméthylique de formule générale I

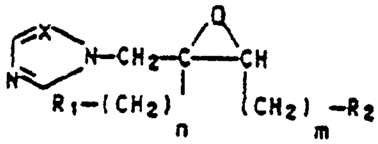

I,

dans laquelle $R_1$ et $R_2$ représentent des groupes alkyle en $C_1$-$C_5$, naphtyle, biphényle, dioxannyle ou phényle, ces groupes pouvant eux-mêmes être éventuellement substitués par des halogènes, des groupes nitro, phénoxy, alkyle, alcoxy amino ou halogénoalkyle contenant chacun 1 à 4 atomes de carbone,
m et n sont des nombres entiers allant de 1 à 5 ou sont égaux à 0, sous réserve que la somme m + n est égale à 1 ou a une valeur supérieure à 1,
X représente CH ou N, ou l'un de ses sels formés par addition avec un acide ou complexes métalliques tolérés par les végétaux.

4. Procédé pour combattre les mycètes, caractérisé en ce que l'on fait agir sur les mycètes ou sur les matières, surfaces, plantes ou semences menacées d'une attaque par les mycètes une quantité fongicide efficace d'un dérivé azolylméthylique de formule générale I

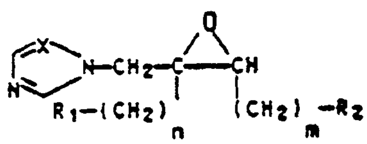

I,

dans laquelle $R_1$ et $R_2$ représentent des groupes alkyle en $C_1$-$C_5$, naphtyle, biphényle, dioxannyle ou phényle, ces groupes pouvant eux-mêmes être éventuellement substitués par des halogènes, des groupes nitro, phénoxy, alkyle, alcoxy, amino ou halogénoalkyle contenant chacun 1 à 4 atomes de carbone,
m et n sont des nombres entiers allant de 1 à 5 ou sont égaux à 0 sous réserve que la somme m + n est égale à 1 ou supérieure à 1,
X représente CH ou N, ou l'un de ses sels formés par addition avec un acide ou complexes métalliques tolérés par les végétaux.

5. Composé selon la revendication 1, caractérisé en ce que $R_1$ représente un groupe tert-butyle, $R_2$ un groupe 4-chlorophényle, n est égal à 1, m à 0 et X représente N.

6. Composé selon la revendication 1, caractérisé en ce que $R_1$ représente un groupe 4-fluorophényle, $R_2$ un groupe tert-butyle, n est égal à 1, m est égal à 1 et X représente N.

7. Composé selon la revendication 1, caractérisé en ce que $R_1$ représente un groupe tert-butyle, $R_2$ un groupe 4-chlorophényle, n est égal à 1, m est égal à 1 et X représente N.

8. Composé de formule

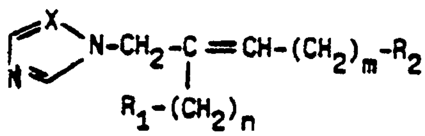

dans laquelle $R_1$ et $R_2$ représentent des groupes alkyle en $C_1$-$C_5$, naphtyle, biphényle, dioxannyle ou phényle, ces groupes pouvant eux-mêmes être substitués éventuellement par des halogènes, des groupes nitro, phénoxy, alkyle, alcoxy, amino ou halogénoalkyle contenant chacun 1 à 4 atomes de carbone,
m et n sont des nombres entiers allant de 1 à 5 ou sont égaux à 0, sous réserve que la somme m + n est égale ou supérieure à 1,
X représente CH ou N, et ses sels formés par addition avec des acides tolérés par les végétaux.

9. Composé selon la revendication 8, caractérisé en ce que $R_1$ représente un groupe tert-butyle, $R_2$ un groupe 4-chlorophényle, n est égal à 1, m est égal à 0 et X représente N, ce composé étant à l'état d'isomère Z.